# EUROPÄISCHE PATENTSCHRIFT

(11) **EP 3 984 445 B1**
(45) Veröffentlichungstag und Bekanntmachung des Hinweises auf die Patenterteilung: **11.09.2024**
(21) Anmeldenummer: 20202483.2
(22) Anmeldetag: 19.10.2020
(51) Int. Cl.: A61B 5/00, A61B 18/00, A61B 18/14, A61B 18/12

(54) **ELEKTROCHIRURGISCHES INSTRUMENT UND ELEKTROCHIRURGISCHE VORRICHTUNG**
ELECTROSURGICAL INSTRUMENT AND ELECTROSURGICAL DEVICE
INSTRUMENT ÉLECTRO-CHIRURGICAL ET DISPOSITIF ÉLECTRO-CHIRURGICAL

(43) Veröffentlichungstag der Anmeldung: 20.04.2022
(73) Patentinhaber: Erbe Elektromedizin GmbH, 72072 Tübingen (DE)
(72) Erfinder: REITERER, Markus, 2620 Loipersbach (AT); FISCHER, Klaus, 72202 Nagold (DE); BRODBECK, Achim, 72555 Metzingen (DE); MÜLLER, Marc, 72074 Tübingen (DE)
(74) Vertreter: Rüger Abel Patentanwälte PartGmbB

(56) Entgegenhaltungen:
- EP-A1- 3 545 888
- US-A- 5 435 805
- US-A1- 2012 289 954

## Beschreibung

Die Erfindung betrifft ein elektrochirurgisches Instrument, die über ein Versorgungsgerät gespeist werden kann, insbesondere mit elektrischer Energie und einem Spülfluid. Das Versorgungsgerät kann außerdem weitere Einrichtungen oder Einheiten aufweisen, die elektrisch und/oder fluidisch und/oder optisch mit dem Instrument verbunden werden können. Die Erfindung betrifft auch eine elektrochirurgische Vorrichtung aufweisend ein elektrochirurgisches Instrument, das an ein Versorgungsgerät angeschlossen ist.

EP 2 815 713 A1 beschreibt ein elektrochirurgisches Instrument aufweisend eine Lichtleiteinrichtung sowie wenigstens eine Elektrode. Mittels der Elektrode kann biologisches Gewebe behandelt werden, beispielsweise zur Dissektion oder Koagulation des Gewebes. Das dabei durch Funkenbildung zwischen der Elektrode und dem Gewebe entstehende Licht kann über ein Lichteintrittsfenster aufgenommen und über die Lichtleiteinrichtung an eine Lichtanalyseeinheit weitergeleitet werden. Basierend auf einer Lichtanalyse kann dann die Art des Gewebes erkannt werden, das mittels der wenigstens einen Elektrode behandelt wird. Zur Aufnahme des Lichts wird an dem Instrument ein Fluidkörper gebildet. Dieser Fluidkörper kann durch einen Fluidkanal und Fluidaustrittsöffnungen am distalen Ende während des Betriebs des Instruments erneuert werden.

Ein ähnliches elektrochirurgisches Instrument ist in EP 3 517 028 A1 beschrieben.

Aus US 2012/0289954 A1 ist ein elektrochirurgisches Instrument in Form eines Katethers bekannt, das einen Mikroplasmakopf an seinem distalen Ende aufweist. Der Mikroplasmakopf weist eine gasumspülte Elektrode auf, um ein Plasma zu generieren. Ablationsprodukte und verbrauchtes Gas werden aus dem Lumen zwischen einem Gaszufuhrkanal und einer Umhüllung abgesaugt. In diesem Lumen der Umhüllung kann bei einem Ausführungsbeispiel ein Lichtwellenleiter angeordnet sein, um das vom Plasma erzeugte Licht aufzunehmen und an ein Spektrometer weiter zu leiten.

Es kann als Aufgabe der vorliegenden Erfindung angesehen werden, ein elektrochirurgisches Instrument zu schaffen, das eine verbesserte Schutzwirkung gegen eine Verschmutzung einer Lichteintrittsfläche einer Lichtleiteinrichtung aufweist.

Diese Aufgabe wird durch ein elektrochirurgisches Instrument mit den Merkmalen des Patentanspruches 1 und eine elektrochirurgische Vorrichtung mit den Merkmalen des Patentanspruches 9 gelöst.

Das erfindungsgemäße elektrochirurgische Instrument kann zur offenchirurgischen oder laparoskopischen Anwendung eingerichtet sein. Zur Verbindung mit einem Versorgungsgerät hat das elektrochirurgische Instrument eine Anschlusseinrichtung. Über die Anschlusseinrichtung kann eine elektrische, optische und fluidische Verbindung zwischen dem elektrochirurgischen Instrument und dem Versorgungsgerät hergestellt werden. Die Anschlusseinrichtung kann beispielsweise einen oder mehrere Stecker oder andere geeignete Verbindungselemente aufweisen, mit denen insbesondere eine lösbare Verbindung zwischen dem Versorgungsgerät und dem elektrochirurgischen Instrument herstellbar ist. Die elektrische Verbindung, die optische Verbindung und die fluidische Verbindung können dabei über einen oder mehrere gemeinsame oder separate Stecker oder andere geeignete Verbindungselemente hergestellt werden.

Das elektrochirurgische Instrument weist außerdem eine Umhüllung mit einer Endöffnung am distalen Ende der Umhüllung auf. Die Umhüllung kann bei einem Ausführungsbeispiel durch ein Gehäuse oder einen Teil des Gehäuses gebildet sein, beispielsweise bei der Ausgestaltung für den offenchirurgischen Einsatz. Ein Teil des Gehäuses kann als Handgriff dienen oder einen Handgriff aufweisen. An dem Gehäuse können außerdem ein oder mehrere Bedienelemente zur manuellen Betätigung vorhanden sein.

Es ist auch möglich, die Umhüllung durch einen flexiblen Schlauch, ein starres Rohr oder eine Kombination aus flexiblen Schlauch- und starren Rohrabschnitten zu bilden, beispielsweise bei einem elektrochirurgischen Instrument für den laparoskopischen Einsatz in Kombination mit einem Endoskop oder den Einsatz mit einem flexiblen Endoskop. Die Umhüllung kann somit abhängig von der Ausgestaltung zumindest abschnittsweise starr oder flexibel sein. Die Umhüllung kann beispielsweise aus Kunststoff und/oder einer Metalllegierung bestehen.

Die Anschlusseinrichtung kann an einem proximalen Ende der Umhüllung angeordnet sein. Die Anschlusseinrichtung kann eine oder mehrere Leitungen und/oder einen oder mehrere Stecker oder ähnliche Verbindungselemente aufweisen.

Das Instrument hat an seinem distalen Ende wenigstens eine Elektrode. Die wenigstens eine Elektrode ist elektrisch mit der Anschlusseinrichtung verbunden und kann daher über die Anschlusseinrichtung mit dem Versorgungsgerät verbunden werden. Die wenigstens eine Elektrode ist abhängig von der Ausführungsform benachbart zur Endöffnung der Umhüllung angeordnet oder ragt in einer Längsrichtung über die Endöffnung hinaus, wobei sich die wenigstens eine Elektrode durch die Endöffnung hindurch erstrecken kann. Die Längsrichtung ist die Richtung, in der sich das Instrument zumindest im Anschluss an die Endöffnung der Umhüllung erstreckt und/oder die Richtung, in die sich die wenigstens eine Elektrode im Anschluss an ihr distales Ende erstreckt. Bei einem Ausführungsbeispiel kann eine Ebene, in der sich die Endöffnung erstreckt, rechtwinkelig zur Längsrichtung ausgerichtet sein.

Das elektrochirurgische Instrument kann als monopolares oder als bipolares oder als multipolares Instrument ausgebildet sein. Bei der Ausgestaltung als monopolares Instrument ist eine einzige Elektrode am Instrument ausreichend. Eine weitere Elektrode, die als Gegenelektrode bezeichnet werden kann, kann separat von dem elektrochirurgischen Instrument ausgebildet und mit dem Versorgungsgerät verbunden werden. Wenn das elektrochirurgische Instrument als bipolares Instrument ausgestaltet ist, sind wenigstens zwei Elektroden vorhanden, zwischen denen beim Betrieb eine Spannung anliegen kann.

Das elektrochirurgische Instrument hat außerdem eine optisch mit der Anschlusseinrichtung verbundene Lichtleiteinrichtung. An ihrem distalen Ende hat die Lichtleiteinrichtung eine Lichteintrittsfläche, die innerhalb der Umhüllung angeordnet ist und die im Wesentlichen zur Endöffnung und/oder zu der wenigstens einen Elektrode hin ausgerichtet ist. Die Lichteintrittsfläche ist derart angeordnet und orientiert, dass bei einer Funkenbildung an der wenigstens einen Elektrode entstehendes Licht durch die Lichteintrittsfläche einfallen und über die Lichtleiteinrichtung optisch zur Anschlusseinrichtung weitergeleitet werden kann. Das aufgenommene Licht kann über die Anschlusseinrichtung an das Versorgungsgerät weitergeleitet werden und dort beispielsweise in einer Lichtanalyseeinheit analysiert werden. Wenn die Lichtanalyseeinheit bei einem Ausführungsbeispiel Bestandteil des elektrochirurgischen Instruments ist, ist die Lichtleiteinrichtung mit der Lichtanalyseeinheit direkt verbunden und eine optische Verbindung mit der Anschlusseinrichtung kann entfallen.

Das elektrochirurgische Instrument hat außerdem einen fluidisch mit der Anschlusseinrichtung verbundenen Spülkanal, der vorzugsweise innerhalb der Umhüllung verläuft. Der Spülkanal hat an seinem distalen Ende eine Austrittsöffnung, die in einen von der Umhüllung begrenzten Strömungsraum mündet. Die Austrittsöffnung ist in Längsrichtung mit Abstand zur Endöffnung angeordnet. In Längsrichtung gegenüberliegend mündet die Endöffnung der Umhüllung in den Strömungsraum. Der Strömungsraum ist somit in Längsrichtung betrachtet zwischen der Austrittsöffnung und der Endöffnung angeordnet. Aus der Austrittsöffnung strömendes Spülfluid bildet im Strömungsraum eine Spülfluidströmung. Vorzugsweise ist die Spülfluidströmung zumindest im Wesentlichen von der Austrittsöffnung weg zur Endöffnung hin gerichtet. Mittels der Spülfluidströmung im Strömungsraum kann das Eindringen von Verunreinigungen, die die Lichteintrittsfläche verunreinigen könnten, vermieden oder zumindest reduziert werden.

Das elektrochirurgische Instrument weist außerdem einen fluidisch mit der Anschlusseinrichtung verbundenen Absaugkanal auf, der vorzugsweise in der Umhüllung angeordnet ist. Der Absaugkanal hat eine in den Strömungsraum mündende Eintrittsöffnung. Über die Eintrittsöffnung und den Absaugkanal kann zumindest ein Teil des Spülfluids aus dem Strömungsraum abgeführt werden. Vorzugsweise ist der Absaugkanal derart dimensioniert, dass im Wesentlichen das gesamte Spülfluid aus dem Strömungsraum abgeführt werden kann, bevor das Spülfluid über die Endöffnung aus dem Strömungsraum austritt.

Somit kann beispielsweise die Menge des Spülfluids eingestellt werden, das die Endöffnung verlässt und auf das zu behandelnde biologische Gewebe auftrifft. Der überwiegende Anteil des in den Strömungsraum eintretenden Spülfluids oder das gesamte in den Strömungsraum eintretende Spülfluid kann über den Absaugkanal abgeführt werden, bevor das Spülfluid aus der Endöffnung austritt. Es hat sich gezeigt, dass dadurch die Verschmutzung der Optik und insbesondere der Lichteintrittsfläche der Lichtleiteinrichtung erheblich verringert werden kann, was zu einer besseren Analyse des aktuell behandelten biologischen Gewebes führt. Der Volumenstrom des Spülfluids kann gegenüber früheren Instrumenten deutlich erhöht werden und ist vorzugsweise größer als 1 l/min. und kann beispielsweise etwa bis zu 8 l/min. betragen.

Es ist möglich, mehrere Absaugkanäle mit jeweils einer Eintrittsöffnung bereitzustellen. Zusätzlich oder alternativ ist es auch möglich, mehrere Spülkanäle mit jeweils einer Austrittsöffnung bereitzustellen.

Bei einer Ausführungsform kann die Austrittsöffnung des Spülkanals bzw. der sich an die Austrittsöffnung anschließende Abschnitt des Spülkanals derart ausgerichtet sein, dass die Hauptströmungsrichtung parallel zur Längsrichtung oder unter einem spitzen Winkel geneigt zur Längsrichtung in den Strömungsraum eintritt. Die Hauptströmungsrichtung kann durch die Mittelachse definiert sein, die rechtwinklig zur Ebene der Austrittsöffnung ausgerichtet ist.

Die Eintrittsöffnung des Absaugkanals ist in Längsrichtung vorzugsweise mit Abstand zur Endöffnung angeordnet. Es ist vorteilhaft, wenn die Eintrittsöffnung des Absaugkanals in Längsrichtung näher an der Endöffnung angeordnet ist als die Austrittsöffnung des Spülkanals. Alternativ können die Eintrittsöffnung und die Austrittsöffnung in Längsrichtung denselben Abstand von der Endöffnung aufweisen. Bei dieser Ausgestaltung ist es möglich, dass die Eintrittsöffnung und die Austrittsöffnung in einer gemeinsamen Ebene angeordnet sind.

Wie bereits erläutert, kann es vorteilhaft sein, wenn das elektrochirurgische Instrument derart dimensioniert ist oder betrieben wird, dass kein Spülfluid der Spülfluidströmung durch die Endöffnung aus dem Strömungsraum austritt. Dies kann beispielsweise dadurch erreicht werden, dass der Volumenstrom des Spülfluids durch den Spülkanal maximal so groß ist wie der Volumenstrom der Strömung durch den Absaugkanal. Zusätzlich kann auch die Geometrie des Strömungsraums und/oder die Anordnung und Ausrichtung der Austrittsöffnung des Spülkanals und/oder die Anordnung und Ausrichtung der Eintrittsöffnung des Absaugkanals die Strömungsrichtung der Spülfluidströmung im Strömungsraum derart gewählt werden, dass das Eintreten des Spülfluids in den Absaugkanal erleichtert wird. Beispielsweise kann sich der Strömungsraum zur Endöffnung hin verjüngen. Zusätzlich oder alternativ können die Austrittsöffnung und die Eintrittsöffnung derart angeordnet und orientiert sein, dass die Spülfluidströmung eine Strömungsrichtungskomponente von der Austrittsöffnung zur Eintrittsöffnung hin aufweist.

Es ist außerdem vorteilhaft, wenn die Lichteintrittsfläche der Lichtleiteinrichtung in Längsrichtung einen größeren Abstand von der Endöffnung aufweist als die Austrittsöffnung des Spülkanals. Dadurch lässt sich der Schutz der Lichteintrittsfläche weiter verbessern.

Die Lichteintrittsfläche bzw. der sich an die Lichteintrittsfläche anschließende distale Endabschnitt der Lichtleiteinrichtung ist im Spülkanal angeordnet, so dass die Lichteintrittsfläche bzw. der sich anschließende distale Endabschnitt der Lichtleiteinrichtung von Spülfluid umströmt wird, insbesondere bevor das Spülfluid die Austrittsöffnung verlässt. Er kann unmittelbar benachbart zur Austrittsöffnung angeordnet sein. Auch durch diese Maßnahme ist eine verbesserte Schutzwirkung gegen Verschmutzung der Lichteintrittsfläche erreicht.

Es kann vorteilhaft sein, wenn neben dem Absaugkanal wenigstens ein elektrisch mit der Anschlusseinrichtung verbundener Sensor vorhanden ist. Mittels des wenigstens einen Sensors kann ein Parameter der Strömung bzw. des Fluids im Absaugkanal erfasst werden, wie beispielsweise ein Druck, ein Volumenstrom, eine Strömungsgeschwindigkeit oder ein anderer physikalischer Parameter oder eine beliebige Kombination mehrerer der genannten Parameter.

Eine erfindungsgemäße elektrochirurgische Vorrichtung weist ein elektrochirurgisches Instrument auf, das einem der vorstehend erläuterten Ausführungsbeispiele entspricht. Mittels der Anschlusseinrichtung ist das elektrochirurgische Instrument an ein Versorgungsgerät angeschlossen. Eine Lichtanalyseeinheit ist optisch mit der Lichtleiteinrichtung bzw. der Lichteintrittsfläche verbunden und dazu eingerichtet, eine Analyse des empfangenen Lichts durchzuführen, insbesondere eine Spektralanalyse. Die Lichtanalyseeinheit kann Bestandteil des elektrochirurgischen Instruments oder des Versorgungsgeräts sein oder separat dazu ausgebildet sein. Wenn die Lichtanalyseeinheit nicht Bestandteil des elektrochirurgischen Instruments ist, ist die Lichtanalyseeinheit optisch mit der Anschlusseinrichtung verbunden, so dass über die Anschlusseinrichtung das Licht an die Lichtanalyseeinheit weitergeleitet werden kann.

Das Versorgungsgerät hat eine Spannungsquelle, die elektrisch mit der wenigstens einen Elektrode des elektrochirurgischen Instruments verbunden ist. Dadurch kann ein elektrisches Spannungspotential, insbesondere ein hochfrequentes Wechselspannungspotential an die wenigstens eine Elektrode angelegt werden.

Außerdem weist das Versorgungsgerät eine Spülfluidquelle auf, die fluidisch mit dem Spülkanal verbunden ist sowie eine Absaugeinheit die fluidisch mit dem Absaugkanal verbunden ist. Mittels der Spülfluidquelle kann dem Spülfluidkanal Spülfluid zugeführt und dadurch die Spülfluidströmung erzeugt werden. Mittels der Absaugeinheit kann eine Absaugströmung durch den Absaugkanal erzeugt werden, um zumindest einen Teil des Spülfluids oder das gesamte Spülfluid aus dem Strömungsraum abzuführen.

Dabei kann der Volumenstrom des Spülfluids und der Volumenstrom der Absaugströmung angepasst an die Anwendung eingestellt werden, wobei der Volumenstrom der Absaugströmung mindestens so groß ist wie der Volumenstrom des Spülfluids und vorzugsweise größer. Bevorzugt wird bzw. werden der Volumenstrom der Absaugströmung und/oder eine Absaugleistung der Absaugeinheit derart eingestellt, dass kein Spülfluid oder lediglich ein geringer Teil des Spülfluids aus der Endöffnung der Umhüllung austritt.

Mittels der Absaugeinheit kann auch eine Absaugleistung und/oder ein Volumenstrom der Absaugströmung eingestellt werden, so dass Rauchgase oder andere Gase, die bei der Behandlung von Gewebe mittels des elektrochirurgischen Instruments entstehen, über die Endöffnung, die Eintrittsöffnung und den Absaugkanal abgesaugt werden.

Während des Betriebs der elektrochirurgischen Vorrichtung wird ein Spannungspotential an die wenigstens eine Elektrode angelegt. Spülfluid wird über den Spülkanal in den Strömungsraum geleitet und zumindest ein Teil davon über die Eintrittsöffnung und den Absaugkanal aus dem Strömungsraum abgeführt. Während der Behandlung des Gewebes, beispielsweise der Dissektion oder der Koagulation des Gewebes, entsteht an der wenigstens einen Elektrode ein Funken, der Licht emittiert. Das Lichtspektrum ist charakteristisch für die Art des behandelten Gewebes. Das Licht kann über die Lichteintrittsfläche einfallen und über die Lichtleiteinrichtung an die Lichtanalyseeinheit weitergeleitet werden, so dass eine Auswertung über die Art des behandelten Gewebes in der Lichtanalyseeinheit möglich ist. Das Ergebnis der Analyse kann dem Operateur optisch und/oder akustisch und/oder haptisch angezeigt werden.

Bei allen Ausführungsformen der Erfindung kann das Spülfluid gasförmig oder flüssig sein. Als Spülfluid kann beispielsweise, Kohlenstoffdioxid oder Luft oder Argon oder eine beliebige Kombination davon verwendet werden. Insbesondere eignet sich Argon zur endoskopischen Verwendung des Instruments und Kohlenstoffdioxid und/oder Luft zur offenchirurgischen Verwendung des Instruments.

Vorteilhafte Ausgestaltungen der Erfindung ergeben sich aus den abhängigen Patentansprüchen, der Beschreibung und den Zeichnungen. Nachfolgend werden bevorzugte Ausführungsbeispiele der Erfindung anhand der beigefügten Zeichnungen erläutert. Die Zeichnungen umfassen folgende Figuren:
Figur 1 ein Blockschaltbild einer elektrochirurgischen Vorrichtung aufweisend ein elektrochirurgisches Instrument sowie ein Versorgungsgerät,
Figur 2 eine perspektivische Darstellung eines Ausführungsbeispiels eines elektrochirurgischen Instruments,
Figuren 3 und 4 jeweils einen sich an das distale Ende des elektrochirurgischen Instruments anschließenden Endabschnitts eines Ausführungsbeispiels des elektrochirurgischen Instruments in einer schematischen Querschnittsdarstellung,
Figur 5 einen sich an das distale Ende des elektrochirurgischen Instruments anschließenden Endabschnitt eines abgewandelten Ausführungsbeispiels und
Figur 6 einen sich an das distale Ende des elektrochirurgischen Instruments anschließenden Endabschnitts eines weiteren Ausführungsbeispiels des elektrochirurgischen Instruments in einer schematischen Querschnittsdarstellung.

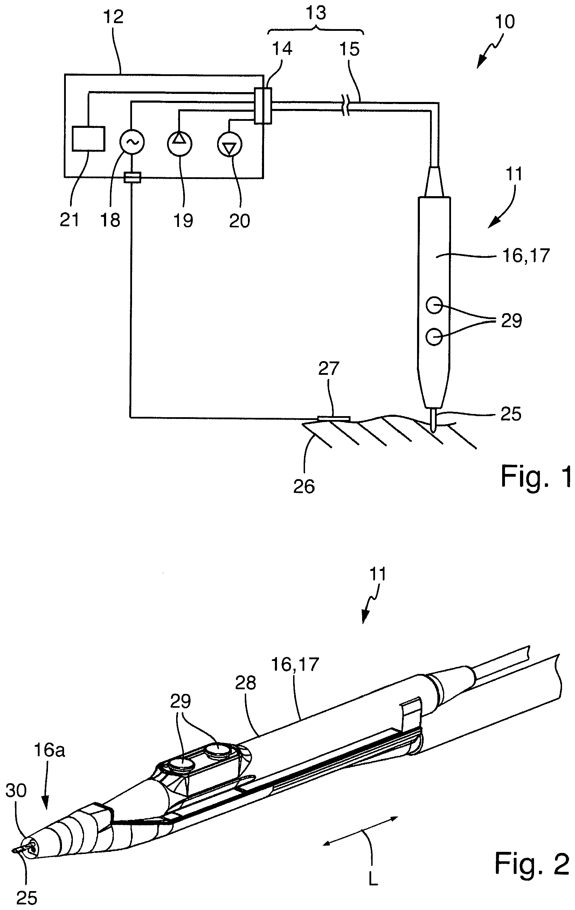

In Figur 1 ist nach Art eines Blockschaltbilds eine elektrochirurgische Vorrichtung 10 veranschaulicht. Die elektrochirurgische Vorrichtung 10 weist ein elektrochirurgisches Instrument 11 und ein Versorgungsgerät 12 auf. Das elektrochirurgische Instrument 11 hat eine Anschlusseinrichtung 13, die dazu eingerichtet ist, eine elektrische und fluidische und beim hier dargestellten Ausführungsbeispiel zusätzlich eine optische Verbindung zwischen dem elektrochirurgischen Instrument 11 und dem Versorgungsgerät 12 herzustellen. Hierzu kann die Anschlusseinrichtung wenigstens ein Verbindungselement 14 aufweisen, mittels dem eine vorzugsweise lösbare Verbindung mit dem Versorgungsgerät 12 herstellbar ist. Das wenigstens eine Verbindungselement 14 kann beispielsweise ein Anschlussstecker der Anschlusseinrichtung 13 sein.

Die Anschlusseinrichtung 13 weist beim Ausführungsbeispiel außerdem mehrere Leitungen 15 auf, wobei die Leitungen 15 fluidische Leitungen, wenigstens eine elektrische Leitung und optional wenigstens eine optische Leitung enthalten. Die Leitungen 15 erstrecken sich von dem wenigstens einen Verbindungselement 14 in eine Umhüllung 17 des elektrochirurgischen Instruments 11. Beim Ausführungsbeispiel gemäß der Figuren 1-4 ist die Umhüllung 17 von einem Gehäuse 16 gebildet.

In Abwandlung zum dargestellten Ausführungsbeispiel könnten auch mehrere separate Verbindungselemente 14 vorhanden sein, beispielsweise ein Verbindungselement 14 zum Anschluss der fluidischen Leitungen 15, ein Verbindungselement 14 zum Anschluss der wenigstens einen elektrischen Leitung und optional ein Verbindungselement 14 zum Anschluss einer optischen Leitung 15.

Das Versorgungsgerät 12 ist dazu eingerichtet, die für den Betrieb des elektrochirurgischen Instruments 11 erforderliche Fluide zuzuführen, elektrische Energie für den Betrieb bereitzustellen sowie Fluide abzuführen. Hierzu weist das Versorgungsgerät 12 beim Ausführungsbeispiel eine Spannungsquelle 18 auf, mittels der eine elektrische Spannung, insbesondere eine Hochfrequenz-Wechselspannung bereitgestellt werden kann. Das Versorgungsgerät 12 hat außerdem eine Spülfluidquelle 19 zur Versorgung des elektrochirurgischen Instruments 11 mit einem Spülfluid sowie eine Absaugeinheit 20. Mittels der Absaugeinheit 20 können Fluide aus dem distalen Endbereich des elektrochirurgischen Instruments 11 abgeführt werden.

Die elektrochirurgische Vorrichtung 10 weist außerdem eine Lichtanalyseeinheit 21 auf, die beim Ausführungsbeispiel außerhalb des Gehäuses 16 des elektrochirurgischen Instruments 11 angeordnet ist. Sie kann Bestandteil des Versorgungsgeräts 12 sein, wie es in Figur 1 veranschaulicht ist. Alternativ dazu kann die Lichtanalyseeinheit 21 auch außerhalb des Versorgungsgeräts 12 als separate Einheit angeordnet sein.

Das elektrochirurgische Instrument 11 hat wenigstens eine Elektrode 25, die elektrisch mit der Anschlusseinrichtung 13 verbunden ist. Über die Anschlusseinrichtung 13 kann mittels der Spannungsquelle 18 ein Spannungspotential und insbesondere ein Hochfrequenz-Wechselspannungspotential an die Elektrode 25 angelegt werden. In Figur 1 ist eine Ausführungsform des elektrochirurgischen Instruments 11 in Form eines monopolaren Instruments veranschaulicht, das lediglich eine Elektrode 25 aufweist. Um bei der Behandlung von biologischem Gewebe 26 mittels des elektrochirurgischen Instruments 11 einen Stromkreis zu schließen, ist eine separate Gegenelektrode 27 elektrisch an das Versorgungsgerät 12 und insbesondere die Spannungsquelle 18 angeschlossen, um einen geschlossenen Stromkreis über das zu behandelnde biologische Gewebe 26 erreichen zu können. Die Gegenelektrode 27 kann hierfür am Patienten angebracht werden.

In Abwandlung zu dem in Figur 1 dargestellten Ausführungsbeispiel kann das elektrochirurgische Instrument 11 auch als bipolares oder multipolares Instrument mit wenigstens zwei Elektroden 25 ausgebildet sein, so dass auf eine separate Gegenelektrode 27 verzichtet werden kann. Der Stromkreis kann dann über die wenigstens zwei Elektroden 25 des elektrochirurgischen Instruments 11 und über das zu behandelnde biologische Gewebe 26 geschlossen werden.

Bei dem in den Figuren 1-4 veranschaulichten Ausführungsbeispiel ist das elektrochirurgische Instrument 11 mit einem Handgriff 28 ausgestattet, mittels dem ein Operateur das elektrochirurgische Instrument 11 hält bzw. führt. Der Handgriff 28 kann, wie beim Ausführungsbeispiel, durch einen Teil des Gehäuses 16 gebildet sein oder am Gehäuse 16 angebracht sein. In der Nähe des Handgriffs 28 kann an dem Gehäuse 16 außerdem wenigstens ein Bedienelement 29 vorhanden sein, beispielsweise um das Versorgungsgerät 12 anzusteuern. Über das wenigstens eine Bedienelement 29 kann beispielsweise ein gewünschtes Spannungspotential an die Elektrode 25 angelegt werden und/oder das Zuführen eines Spülfluids mittels der Spülfluidquelle 19 gesteuert werden und/oder das Absaugen mittels der Absaugeinheit 20 gesteuert werden.

Die Umhüllung 17 und beispielsgemäß das Gehäuse 16 hat an ihrem distalen Ende eine Endöffnung 30. Bei dem Ausführungsbeispiel gemäß der Figuren 1 bis 4 ragt die wenigstens eine Elektrode 25 in einer Längsrichtung L über das distale Ende des Gehäuses 16 bzw. der Umhüllung 17 hinaus und kann sich durch die Endöffnung 30 hindurch erstrecken. Die Längsrichtung L ist insbesondere durch die Richtung definiert, in der sich die Elektrode 25 ausgehend von ihrem distalen Ende erstreckt und/oder die Richtung, in der sich die Längsachse der Umhüllung 17 bzw. des Gehäuses 16 erstreckt. Die Längsrichtung L ist beim Ausführungsbeispiel rechtwinklig zu einer Ebene orientiert, in der sich die Endöffnung 30 erstreckt.

Wie es in Figur 2 veranschaulicht ist, kann das Gehäuse 16 aus mehreren zusammengefügten Teilen bestehen. Alternativ hierzu kann das Gehäuse 16 auch einteilig ausgebildet sein.

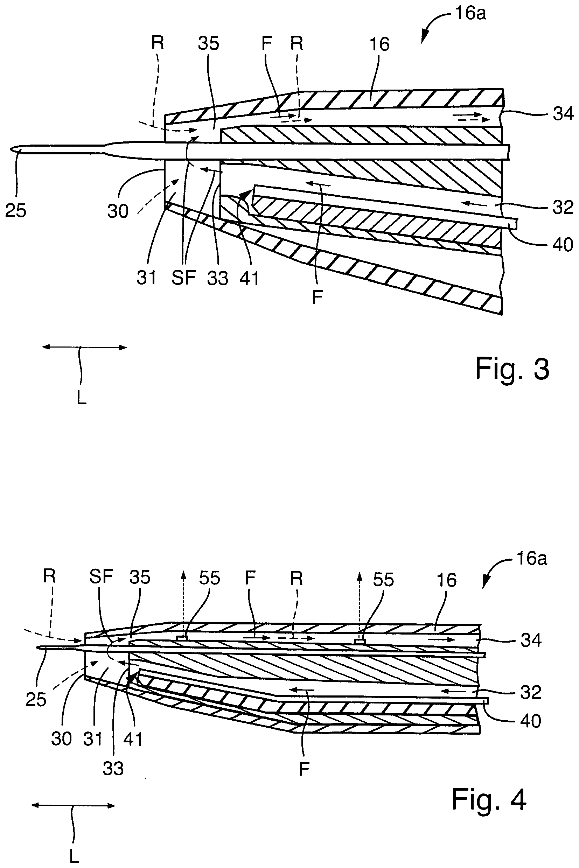

In den Figuren 3 und 4 ist der sich an die Endöffnung 30 anschließende distale Endabschnitt 16a des Gehäuses 16 vergrößert in einem schematischen Querschnitt veranschaulicht. Das Gehäuse 16 begrenzt unmittelbar im Anschluss an die am distalen Ende vorhandene Endöffnung 30 einen Strömungsraum 31. Ein Spülkanal 32, der vorzugsweise innerhalb des Gehäuses 16 bzw. der Umhüllung 17 verläuft, hat eine Austrittsöffnung 33, die unmittelbar an den Strömungsraum 31 angrenzt. An der Austrittsöffnung 33 mündet der Spülkanal 32 somit in den Strömungsraum 31. Das an der Austrittsöffnung 33 austretende Spülfluid F erzeugt im Strömungsraum 31 eine Spülfluidströmung SF.

Die Austrittsöffnung 33 und/oder der sich daran anschließende Abschnitt des Spülkanals 32 sind beispielsgemäß unter einem spitzen Winkel zur Längsrichtung L ausgerichtet. Die Hauptströmungsrichtung der Spülfluidströmung SF im Anschluss an die Austrittsöffnung 33 entspricht beispielsgemäß der Längsmittelachse des sich an die Austrittsöffnung 33 anschließenden Abschnitts des Spülkanals 32, die unter einem Winkel von beispielsweise maximal 30° oder maximal 20° oder maximal 10° relativ zur Längsrichtung L ausgerichtet sein kann. Bei anderen Ausführungsbeispielen kann die Hauptströmungsrichtung auch parallel zur Längsrichtung L orientiert sein.

Das elektrochirurgische Instrument 11 hat außerdem einen Absaugkanal 34, der über eine Eintrittsöffnung 35 unmittelbar in den Strömungsraum 31 mündet. Über den Absaugkanal 34 kann Fluid aus dem Strömungsraum 31 weggeleitet werden, insbesondere Spülfluid F. Außerdem können über den Absaugkanal 34 Rauchgase R abgesaugt werden, die während der Behandlung von biologischem Gewebe 26 entstehen. Solche Rauchgase R können über die Endöffnung 30 in den Strömungsraum 31 eingesaugt und weiter über die Eintrittsöffnung 35 in den Absaugkanal 34 gelangen.

Beim Ausführungsbeispiel ist die Elektrode 25 rechtwinklig zur Längsrichtung L betrachtet zwischen der Austrittsöffnung 33 des Spülkanals 32 und der Eintrittsöffnung 35 des Absaugkanals 34 angeordnet.

In betriebsbereitem Zustand des elektrochirurgischen Instruments 11 ist der Absaugkanal 34 fluidisch mit der Absaugeinheit 20 verbunden und der Spülkanal 32 ist fluidisch mit der Spülfluidquelle 19 verbunden. Außerdem besteht eine elektrische Verbindung zwischen der Spannungsquelle 18 und der Elektrode 25.

Das elektrochirurgische Instrument verfügt außerdem über eine Lichtleiteinrichtung 40, die eine dem Strömungsraum 31 bzw. der wenigstens einen Elektrode 25 zugewandte Lichteintrittsfläche 41 aufweist. Die Lichtleiteinrichtung 40 kann beispielsweise durch einen Lichtleiter, wie etwa eine Glasfaser, gebildet sein. Die Lichteintrittsfläche 41 kann durch die Stirnfläche des Lichtleiters gebildet sein. Alternativ dazu kann der Lichtleiter mit einer Optik gekoppelt sein, beispielsweise einer Linse, an der die Lichteintrittsfläche 41 vorhanden ist.

Die Lichteintrittsfläche 41 ist derart ausgerichtet, dass sie Licht empfangen kann, das an der Elektrode 25 beim Bearbeiten des biologischen Gewebes 26 durch Funkenbildung emittiert wird. Dieses Licht wird über die Lichtleiteinrichtung 40 an die Lichtanalyseeinheit 21 zur Auswertung weitergeleitet, insbesondere zur Spektralanalyse. Dadurch kann auf die Art des bearbeiteten Gewebes geschlossen werden.

Die Lichteintrittsfläche 41 ist empfindlich gegen Verschmutzung. Über das in den Strömungsraum 31 eingeleitete Spülfluid F kann eine Verschmutzung der Lichteintrittsfläche 41 verhindert oder zumindest gemindert werden. Beispielsweise können Gewebepartikel, Rauchgaspartikel, Gewebeflüssigkeit oder dergleichen auf die Lichteintrittsfläche 41 gelangen und sich dort ansammeln. Es hat sich gezeigt, dass der Schutz der Lichteintrittsfläche 41 vor Verschmutzung verbessert werden kann, wenn das Spülfluid F zumindest im Wesentlichen innerhalb des Strömungsraums 31 verbleibt und nicht oder lediglich zu einem geringeren Teil durch die Endöffnung 30 austritt. Durch das Auftreffen des Spülfluids F auf das Gewebe 26 können Schmutzpartikel durch die Endöffnung 30 auf die Lichteintrittsfläche 41 geschleudert werden. Dadurch, dass der Anteil des auf das biologische Gewebe 26 auftreffenden Spülfluids F verringert wird, reduziert die Gefahr einer derartigen Verschmutzung der Lichteintrittsfläche 41.

Deswegen ist bei der erfindungsgemäßen Ausgestaltung des elektrochirurgischen Instruments 11 innerhalb der Umhüllung 17 bzw. des Gehäuses 16 der Strömungsraum 31 gebildet, so dass Spülfluid F über den Absaugkanal 34 abgeführt werden kann, bevor es die Umhüllung 17 bzw. das Gehäuse 16 verlässt. Es ist vorteilhaft, wenn im Wesentlichen das gesamte Spülfluid F über den Absaugkanal 34 abgeführt wird, das über den Spülkanal 32 in den Strömungsraum 31 zugeführt wird. Wie erläutert, können zusätzlich auch Rauchgase R über den Absaugkanal 34 von der Behandlungsstelle weg gefördert werden.

Zum Schutz der Lichteintrittsfläche 41 ist die Lichteintrittsfläche 41 und insbesondere der sich daran anschließende Abschnitt der Lichtleiteinrichtung 40 von dem Spülfluid F umströmt, insbesondere bevor das Spülfluid F an der Austrittsöffnung 33 in den Strömungsraum 31 austritt. Hierzu ist der distale Endabschnitt der Lichtleiteinrichtung 40 im Anschluss an die Lichteintrittsfläche 41 unmittelbar benachbart zum Spülkanal 32 oder innerhalb des Spülkanals 32 angeordnet, wie es beispielsweise in den Figuren 3 und 4 veranschaulicht ist. Es ist dabei vorteilhaft, wenn die Lichteintrittsfläche 41 in Längsrichtung L einen größeren Abstand zur Endöffnung 30 aufweist als die Austrittsöffnung 33.

Es ist auch bevorzugt, wenn die Austrittsöffnung 33 in Längsrichtung L einen Abstand zur distal angeordneten Endöffnung 30 aufweist, der mindestens so groß ist, wie der Abstand in Längsrichtung L zwischen der Eintrittsöffnung 35 des Absaugkanals 34 und der Endöffnung 30. Durch diese Anordnung wird das Absaugen des Spülfluids F aus dem Strömungsraum 31 vereinfacht bzw. verbessert.

Beim Betrieb des elektrochirurgischen Instruments 11 ist der Massen- oder Volumenstrom, der mittels der Spülfluidquelle 19 über den Spülkanal 32 in den Strömungsraum 31 zugeführt wird, maximal so groß wie der Massen- oder Volumenstrom der Strömung im Absaugkanal 34, der mittels der Absaugeinheit 20 eingestellt wird. Vorzugsweise ist der Massen- oder Volumenstrom der Strömung im Absaugkanal 34 größer als der Massen- oder Volumenstrom im Spülkanal 32.

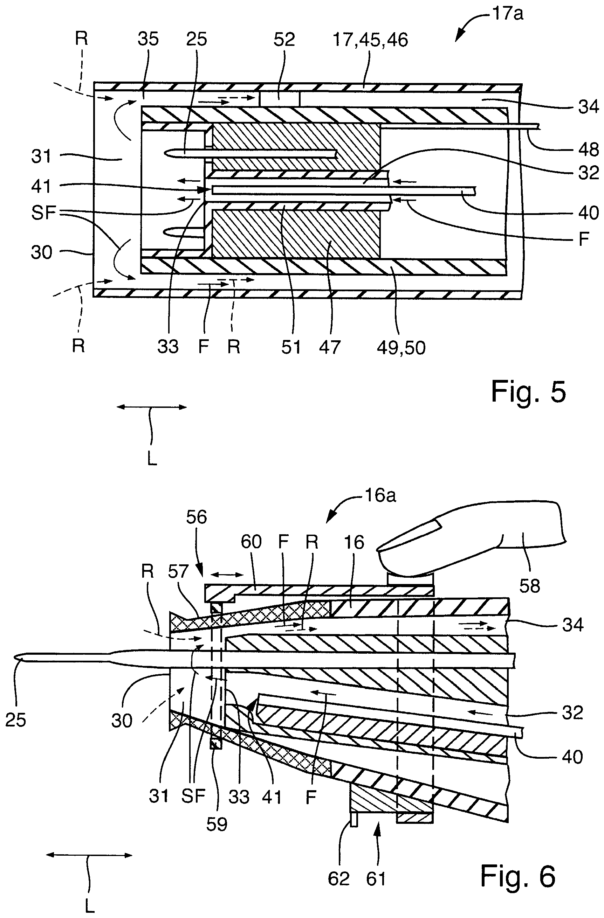

Das bisher beschriebene Ausführungsbeispiel des elektrochirurgischen Instruments 11 ist insbesondere für eine offenchirurgische Anwendung eingerichtet. Ein abgewandeltes Ausführungsbeispiel des elektrochirurgischen Instruments ist in Figur 5 veranschaulicht, das sich insbesondere auch für laparoskopische Eingriffe oder Eingriffe mit einem flexiblen Endoskop bzw. minimalinvasive chirurgische Eingriffe eignet.

Figur 5 zeigt einen schematischen Querschnitt eines distalen Endabschnitts 17a einer Umhüllung 17 des elektrochirurgischen Instruments. Die Umhüllung 17 ist bei diesem Ausführungsbeispiel durch einen flexiblen Schlauch 45 oder ein starres Rohr 46 gebildet. Die Umhüllung 17 kann auch in wenigstens einem Abschnitt als flexibler Schlauch 45 und in wenigstens einem anderen Abschnitt als starres Rohr 46 ausgeführt sein. Die Umhüllung 17 hat beim Ausführungsbeispiel zumindest im distalen Endabschnitt 17a eine hohlzylindrische Gestalt.

Innerhalb der Umhüllung 17 ist ein Elektrodenträger 47 angeordnet, der elektrisch mit wenigstens einer Elektrode 25 und einem elektrischen Leiter 48 verbunden ist. Der elektrische Leiter 48 erstreckt sich innerhalb der Umhüllung 17 und ist mit der Anschlusseinrichtung 13 elektrisch verbunden. Der Elektrodenträger 47 ist beim Ausführungsbeispiel innerhalb eines Innenschlauchs 49 oder eines Innenrohrs 50 angeordnet. Angepasst an die Umhüllung 17 kann ein flexibler Innenschlauch 49 oder ein starres Innenrohr 50 gewählt werden.

Durch den Innenschlauch 49 oder das Innenrohr 50 erstreckt sich der Spülkanal 32, der beispielsweise entlang der Längsachse der Umhüllung 17 verläuft. Die Lichtleiteinrichtung 40 und die Lichteintrittsfläche 41 sind bei diesem Ausführungsbeispiel innerhalb des Spülkanals 32 angeordnet. Im Bereich des distalen Endes des Spülkanals 32 ist benachbart zum Spülkanal 32 der Elektrodenträger 47 angeordnet, der den Spülkanal 32 beispielsweise koaxial umschließen kann. Zwischen dem Elektrodenträger 47 und dem Spülkanal 32 kann eine thermische Abschirmung vorhanden sein, die beispielsweise Bestandteil eines Abschirmelements 51 ist. Ein erster Abschnitt des Abschirmelements 51 umschließt den distalen Endabschnitt des Spülkanals 32 koaxial und bildet beispielsgemäß die Austrittsöffnung 33. Im Anschluss an diesen sich koaxial zum Spülkanal 32 erstreckenden ersten Abschnitt weist das Abschirmelement 51 einen becherförmigen zweiten Abschnitt auf, der innerhalb des Strömungsraums 31 angeordnet ist.

Die wenigstens eine Elektrode 25 erstreckt sich ausgehend vom Elektrodenträger 47 durch das Abschirmelement 51 in den Innenbereich des becherförmigen zweiten Abschnitts des Abschirmelements 51. Die wenigstens eine Elektrode 25 endet mit ihrem distalen Ende vorzugsweise innerhalb des zweiten Abschnitts des Abschirmelements 51. Das distale Ende des Abschirmelements 51 befindet sich beispielsgemäß auf derselben Höhe wie das distale Ende des Innenschlauchs 49 bzw. des Innenrohrs 50. Das distale Ende des Innenschlauchs 49 bzw. des Innenrohrs 50 und/oder das distale Ende des Abschirmelements 51 sind beispielsgemäß in Längsrichtung L mit Abstand zur distal angeordneten Endöffnung 30 innerhalb der Umhüllung 17 angeordnet.

Der Absaugkanal 34 ist beispielsgemäß durch einen Radialabstand zwischen dem Innenschlauch 49 bzw. dem Innenrohr 50 einerseits und der Umhüllung 17 (Schlauch 45 oder Rohr 46) gebildet. In Umfangsrichtung verteilt sind vorzugsweise mehrere Abstandhalter 52 im Absaugkanal 34 angeordnet, um die Relativlage zwischen dem Innenschlauch 49 bzw. dem Innenrohr 50 einerseits und der Umhüllung 17 andererseits zu definieren. Der Innenschlauch 49 bzw. das Innenrohr 50 und die Umhüllung 17 können zumindest abschnittsweise koaxial und/oder zumindest abschnittsweise nicht koaxial zueinander angeordnet sein. Die Strömung durch den Absaugkanal 34 kann an den Abstandhaltern 52 vorbeiströmen. Die Dimension und/oder Form und/oder Position und/oder Oberflächenbeschaffenheit eines oder mehrerer oder aller Abstandhalter 52 kann gewählt werden, um eine Strömungseigenschaft der Strömung durch den Absaugkanal 34 zu beeinflussen. Beispielsweise kann der Volumenstrom durch den Absaugkanal 34 gezielt eingestellt bzw. gedrosselt werden.

Auch bei dem Ausführungsbeispiel gemäß Figur 5 sind die Eintrittsöffnung 35 des Absaugkanals 34 und die Austrittsöffnung 33 des Spülkanals 32 innerhalb der Umhüllung 17 und in Längsrichtung L mit Abstand zur Endöffnung 30 der Umhüllung 17 angeordnet. Wie es in Figur 5 zu erkennen ist, ist der Abstand zwischen der Austrittsöffnung 33 und der Endöffnung 30 größer als der Abstand zwischen der Eintrittsöffnung 35 und der Endöffnung 30.

Die vorstehend beschriebenen Ausführungsbeispiele des elektrochirurgischen Instruments 11 bzw. der elektrochirurgischen Vorrichtung 10 arbeiten bei der Behandlung von biologischem Gewebe 26 wie folgt:

Das elektrochirurgische Instrument 11 wird mittels der Anschlusseinrichtung 13 an das Versorgungsgerät 12 angeschlossen. Falls erforderlich, wird die Gegenelektrode 27 am Patienten befestigt und ebenfalls mit dem Versorgungsgerät 12 verbunden. Wie erläutert, ist bei bipolaren elektrochirurgischen Instrumenten 11 eine separate Gegenelektrode 27 nicht erforderlich.

Über das wenigstens eine Bedienelement 29 steuert der Operateur den Betrieb des elektrochirurgischen Instruments 11 und beispielsweise das Anlegen eines Wechselspannungspotentials an die wenigstens eine Elektrode 25, um das biologische Gewebe 26 zu behandeln, beispielsweise zu schneiden oder zu versiegeln. Dabei entstehen Funken an der Elektrode 25, deren Licht in die Lichteintrittsfläche 41 eintritt und über die Lichtleiteinrichtung 40 an die Lichtanalyseeinheit 21 weitergeleitet wird. Die Lichtanalyseeinheit 21 ist dazu eingerichtet, das empfangene Licht zu analysieren, insbesondere mittels einer Spektralanalyse, um die Art des Gewebes 26 zu ermitteln, das aktuell mittels des elektrochirurgischen Instruments 11 behandelt wird.

Während dieser Behandlung wird mittels der Spülfluidquelle 19 Spülfluid F in den Spülkanal 32 eingeleitet und strömt dort über die Austrittsöffnung 33 in den Strömungsraum 31. Im Anschluss an die Austrittsöffnung 33 bildet sich im Strömungsraum 31 eine Spülfluidströmung SF. Beim Ausführungsbeispiel wird der größte Teil und insbesondere mindestens 75% oder mindestens 80% oder mindestens 90% und weiter vorzugsweise das gesamte Spülfluid F, das in den Strömungsraum 31 einströmt, über den Absaugkanal 34 aus dem Strömungsraum 31 abgeführt. Höchstens ein geringerer Anteil des Spülfluids F kann den Strömungsraum 31 durch die Endöffnung 30 verlassen. Vorzugsweise tritt aus der Endöffnung 30 kein Spülfluid F aus. Das Absaugen des Spülfluids F aus dem Strömungsraum 31 erfolgt mittels der Absaugeinheit 20, die einen entsprechend großen Volumenstrom bzw. Unterdruck und mithin eine entsprechend große Absaugleistung einstellt, um den gewünschten Effekt zu erzielen. Die bei der Behandlung des Gewebes 26 entstehenden Rauchgase R werden durch die Endöffnung 30 und die Eintrittsöffnung 35 ebenfalls in den Absaugkanal 34 eingesaugt und abgeführt.

Bei sämtlichen Ausführungsbeispielen kann in dem Absaugkanal 34 wenigstens ein Sensor 55 angeordnet sein. Der wenigstens eine Sensor 55 ist vorzugsweise elektrisch mit der Anschlusseinrichtung 13 verbunden, so dass ein elektrisches Sensorsignal über die Anschlusseinrichtung 13 an das Versorgungsgerät 12 übermittelt werden kann. Der wenigstens eine Sensor 55 ist dazu eingerichtet, einen Parameter der Strömung innerhalb des Absaugkanals 34 zu erfassen, wie etwa einen Druck, eine Strömungsgeschwindigkeit, einen Volumenstrom, usw. Für die Messung unterschiedlicher Parameter können mehrere verschiedene Sensoren 55 vorhanden sein. Bei dieser Ausgestaltung besteht die Möglichkeit, die Absaugeinheit 20 basierend auf Sollwerten für den wenigstens einen Strömungsparameter zu regeln.

In Figur 6 ist ein distaler Endabschnitt 16a eines weiteren abgewandelten Ausführungsbeispiels des elektrochirurgischen Instruments 11 veranschaulicht. Das elektrochirurgische Instrument 11 ist ähnlich aufgebaut, wie das Ausführungsbeispiel gemäß der Figuren 3 und 4. Nachfolgend werden die Unterschiede gegenüber dem Ausführungsbeispiel der Figuren 3 und 4 erläutert und im Übrigen wird auf die vorstehende Beschreibung verwiesen.

Im Unterschied zu dem ersten Ausführungsbeispiel gemäß der Figuren 3 und 4 hat das Gehäuse 16 einen distalen Endabschnitt 16a, bei dem sich unmittelbar an die Endöffnung 30 ein mittels einer Einstelleinrichtung 56 im Querschnitt veränderbarer Bereich 57 anschließt. Der Bereich 57 des Endabschnitts 16a des Gehäuses 16 kann beispielsweise aus einem flexiblen oder elastischen Material bestehen, das sich unter Einwirkung der Einstelleinrichtung 56 elastisch verformen lässt. Dadurch kann der Strömungsquerschnitt der Endöffnung 30 sowie der Strömungsquerschnitt innerhalb des Bereichs 57 mittels der Einstelleinrichtung 56 eingestellt werden. Die Einstelleinrichtung 56 ist beispielsgemäß manuell bedienbar, beispielsweise mit einem Finger 58.

Zu der Einstelleinrichtung 56 gehört ein in Längsrichtung L verschiebbar gelagerter Einstellring 59, der den Bereich 57 umgibt. Der Bereich 57 weist einen sich ändernden Außendurchmesser und/oder einen sich ändernden Außenumfang auf. Die Position des Einstellrings 59 relativ zum Bereich 57 gibt dessen Außenumfang an dieser Stelle vor. Dadurch wird wiederum der Innenquerschnitt des Bereichs 57 und mithin der Strömungsquerschnitt sowie der Querschnitt der Endöffnung 30 definiert. Anders ausgedrückt wird der Bereich 57 des distalen Endabschnitts 16a radial zur Längsrichtung L betrachtet größer oder kleiner abhängig von der Relativlage zwischen dem Einstellring 59 und dem Bereich 57 in Längsrichtung L.

Der Einstellring 59 ist beim Ausführungsbeispiel an einem in Längsrichtung L verschiebbar am Gehäuse 16 angeordneten Träger 60 angeordnet. Der Träger 60 kann beispielsweise auf einem zylindrischen Lagerteil 61 in Längsrichtung L verschiebbar angeordnet sein. Der Lagerteil 61 kann zumindest teilweise durch das Gehäuse 16 und/oder durch zusätzliche mit dem Gehäuse 16 verbundene Bestandteile gebildet werden. Zumindest ein Teil des Trägers 60 kann den Lagerteil 61 in Umfangsrichtung um die Längsrichtung L teilweise oder vollständig umschließen.

In Richtung zum distalen Ende hin kann an dem Lagerteil 61 ein Anschlag 62 vorhanden sein, um die Bewegung des Trägers 60 in Längsrichtung und mithin die Bewegung des Einstellrings 59 in Längsrichtung L zu begrenzen. Beim Ausführungsbeispiel ist ein einziger Anschlag 62 vorhanden, der die Bewegung des Trägers 60 und des Einstellrings 59 in Richtung distal begrenzt. Zusätzlich oder alternativ kann auch ein weiterer Anschlag vorhanden sein, der die Bewegung in Richtung proximal begrenzt, sofern dies erforderlich ist. Die Bewegung in Richtung proximal wird bei dem in Figur 6 veranschaulichten Ausführungsbeispiel dadurch begrenzt, dass der Bereich 57 an einer Stelle in einen nicht verformbaren, insbesondere nicht elastischen Teil des Gehäuses 16 übergeht, so dass der Einstellring 59 nur höchstens bis zu dem nicht verformbaren Teil des Gehäuses 16 bewegbar ist.

Die Erfindung betrifft ein elektrochirurgisches Instrument 11, das für den Betrieb an ein Versorgungsgerät 12 anschließbar ist. Das elektrochirurgische Instrument 11 hat am distalen Ende eine Endöffnung 30 und trägt im Bereich der Endöffnung 30 wenigstens eine Elektrode 25, an die ein Wechselspannungspotential angelegt werden kann. Unmittelbar im Anschluss an die Endöffnung 30 begrenzt eine Umhüllung 17 des elektrochirurgischen Instruments 11 einen Strömungsraum 31 innerhalb der Umhüllung 17. Mit Abstand zu der Endöffnung 30 mündet ein Spülkanal 32 über eine Austrittsöffnung 33 in den Strömungsraum 31 und ein Absaugkanal 34 mündet über eine Eintrittsöffnung 35 in den Strömungsraum 31. Über den Spülkanal 32 kann ein Spülfluid F in den Strömungsraum 31 eingeführt und über den Absaugkanal 34 abgesaugt werden. Der überwiegende Teil des in den Strömungsraum 31 einströmenden Spülfluids F wird über den Absaugkanal 34 aus dem Strömungsraum 31 abgeführt und kann somit den Strömungsraum 31 nicht über die Endöffnung 30 verlassen. Dadurch kann eine Lichteintrittsfläche 41 einer Lichtleiteinrichtung 40, die innerhalb der Umhüllung 17 angeordnet ist, effektiv vor einer Verschmutzung geschützt werden.

### Bezugszeichenliste:

- 10: elektrochirurgische Vorrichtung
- 11: elektrochirurgisches Instrument
- 12: Versorgungsgerät
- 13: Anschlusseinrichtung
- 14: Verbindungselement
- 15: Leitung
- 16: Gehäuse
- 16a: distaler Endabschnitt des Gehäuses
- 17: Umhüllung
- 17a: distaler Endabschnitt der Umhüllung
- 18: Spannungsquelle
- 19: Spülfluidquelle
- 20: Absaugeinheit
- 21: Lichtanalyseeinheit

- 25: Elektrode
- 26: biologisches Gewebe
- 27: Gegenelektrode
- 28: Handgriff
- 29: Bedienelement

- 30: Endöffnung
- 31: Strömungsraum
- 32: Spülkanal
- 33: Austrittsöffnung
- 34: Absaugkanal
- 35: Eintrittsöffnung

- 40: Lichtleiteinrichtung
- 41: Lichteintrittsfläche

- 45: Schlauch
- 46: Rohr
- 47: Elektrodenträger
- 48: elektrischer Leiter
- 49: Innenschlauch
- 50: Innenrohr
- 51: Abschirmelement
- 52: Abstandhalter

- 55: Sensor
- 56: Einstelleinrichtung
- 57: Bereich des distalen Endabschnitts des Gehäuses
- 58: Finger
- 59: Einstellring
- 60: Träger
- 61: Lagerteil
- 62: Anschlag

- F: Spülfluid
- L: Längsrichtung
- R: Rauchgas
- SF: Spülfluidströmung

## Patentansprüche

1. Elektrochirurgisches Instrument (11) aufweisend:
- eine zur Verbindung mit einem Versorgungsgerät (12) eingerichtete Anschlusseinrichtung (13),
- eine Umhüllung (17) mit einer Endöffnung (30) am distalen Ende der Umhüllung (17),
- wenigstens eine elektrisch mit der Anschlusseinrichtung (13) verbundene Elektrode (25), die benachbart zur Endöffnung (30) angeordnet ist oder in einer Längsrichtung (L) über die Endöffnung (30) hinausragt,
- ein fluidisch mit der Anschlusseinrichtung (13) verbundener Spülkanal (32), der eine Austrittsöffnung (33) aufweist, die in einen von der Umhüllung (17) begrenzten Strömungsraum (31) mündet und die in Längsrichtung (L) mit Abstand zur distal angeordneten Endöffnung (30) angeordnet ist, so dass ein durch den Spülkanal (32) strömendes Spülfluid (F) aus der Austrittsöffnung (33) ausströmt und im Strömungsraum (31) eine Spülfluidströmung (SF) von der Austrittsöffnung (33) in Richtung zur Endöffnung (30) hin erzeugt, und
- ein fluidisch mit der Anschlusseinrichtung (13) verbundener Absaugkanal (34), der eine in den Strömungsraum (31) mündende Eintrittsöffnung (35) aufweist und der dazu eingerichtet ist, zumindest einen Teil des Spülfluids (F) der Spülfluidströmung (SF) über die Eintrittsöffnung (35) aufzunehmen und aus dem Strömungsraum (31) abzuführen,
- eine Lichtleiteinrichtung (40), die eine Lichteintrittsfläche (41) aufweist, **dadurch gekennzeichnet, dass** die Lichtleiteinrichtung (40) 1 innerhalb der Umhüllung (17) angeordnet ist, wobei zumindest der sich an die Lichteintrittsfläche (41) anschließende distale Endabschnitt der Lichtleiteinrichtung (40) im Spülkanal (32) angeordnet ist.

2. Elektrochirurgisches Instrument nach Anspruch 1, wobei die Eintrittsöffnung (35) des Absaugkanals (34) in Längsrichtung (L) mit Abstand zur Endöffnung (30) angeordnet ist.

3. Elektrochirurgisches Instrument nach einem der vorhergehenden Ansprüche, wobei die Eintrittsöffnung (35) des Absaugkanals (34) in Längsrichtung (L) näher an der Endöffnung (30) ist als die Austrittsöffnung (33) des Spülkanals (32) oder wobei die Eintrittsöffnung (35) und die Austrittsöffnung (33) in Längsrichtung (L) denselben Abstand von der Endöffnung (30) aufweisen.

4. Elektrochirurgisches Instrument nach einem der vorhergehenden Ansprüche, wobei die Lichteintrittsfläche (41) der Lichtleiteinrichtung (40) in Längsrichtung (L) weiter entfernt ist von der Endöffnung (30) als die Austrittsöffnung (33).

5. Elektrochirurgisches Instrument nach einem der vorhergehenden Ansprüche, wobei in dem Absaugkanal (34) wenigstens ein elektrisch mit der Anschlusseinrichtung (13) verbundenen Sensor (55) aufweist, der dazu eingerichtet ist einen Druck und/oder einen Strömungsparameter im Absaugkanal (32) zu erfassen.

6. Elektrochirurgisches Instrument nach einem der vorhergehenden Ansprüche, wobei die Umhüllung (17) Teil eines Gehäuses (16) ist, an dem die Anschlusseinrichtung (13) am proximalen Ende angeordnet ist.

7. Elektrochirurgisches Instrument nach einem der Ansprüche 1 bis 5, das als flexibel-endoskopisches oder laparoskopisches Instrument ausgebildet ist.

8. Elektrochirurgisches Instrument nach Anspruch 7, wobei die Umhüllung (17) einen flexiblen Schlauch (45) und/oder ein starres Rohr (46) aufweist.

9. Elektrochirurgische Vorrichtung (10) aufweisend ein elektrochirurgisches Instrument (11) nach einem der vorhergehenden Ansprüche, ein Versorgungsgerät (12), das mittels der Anschlusseinrichtung (13) mit dem elektrochirurgischen Instrument (11) verbunden ist, und eine Lichtanalyseeinheit (21),
wobei die Lichtanalyseeinheit (21) vorhanden ist, die optisch mit der Lichtleiteinrichtung (40) verbunden und dazu eingerichtet ist, eine Analyse des über die Lichteintrittsfläche (41) empfangenen Lichts durchzuführen,
wobei das Versorgungsgerät (12) eine Spannungsquelle (19) aufweist, die elektrisch mit der wenigstens einen Elektrode (25) des elektrochirurgischen Instruments (11) verbunden ist, um eine elektrisches Spannungspotential an die wenigstens einen Elektrode (25) anzulegen,
wobei das Versorgungsgerät (12) eine Spülfluidquelle (19) aufweist, die fluidisch mit dem Spülkanal (32) verbunden ist, um das Spülfluid (F) dem Spülkanal (32) zuzuführen,
und wobei das Versorgungsgerät (12) eine Absaugeinheit (20) aufweist, die fluidisch mit dem Absaugkanal (34) des elektrochirurgischen Instruments (11) verbunden ist, um zumindest einen Teil des Spülfluids (F) der Spülfluidströmung (SF) über die Eintrittsöffnung (35) und den Absaugkanal (34) aus dem Strömungsraum (31) abzuführen.

10. Elektrochirurgische Vorrichtung nach Anspruch 9, wobei das Versorgungsgerät (12) dazu eingerichtet ist, mittels der Spülfluidquelle (19) einen Volumenstrom des Spülfluids (F) einzustellen und mittels der Absaugeinheit (20) einen Volumenstrom der Absaugströmung einzustellen, wobei der Volumenstrom der Absaugströmung mindestens so groß ist wie der Volumenstrom des Spülfluids.

11. Elektrochirurgische Vorrichtung nach Anspruch 9 oder 10, wobei das Versorgungsgerät (12) dazu eingerichtet ist, mittels der Absaugeinheit (20) einen Volumenstrom der Absaugströmung und/oder eine Absaugleistung derart einzustellen, dass kein Spülfluid (F) aus der Endöffnung (30) der Umhüllung (17) austritt.

12. Elektrochirurgische Vorrichtung nach einem der Ansprüche 9 bis 11, wobei das Versorgungsgerät (12) dazu eingerichtet ist, mittels der Absaugeinheit (20) einen Volumenstrom der Absaugströmung und/oder eine Absaugleistung derart einzustellen, dass beim Behandeln von Gewebe (26) entstehende Rauchgase durch die Endöffnung (30) der Umhüllung (17) angesaugt werden.

## Claims

1. Electrosurgical instrument (11) comprising:
- a connection device (13) configured for a connection with a supply apparatus (12),
- a liner (17) having an end opening (30) at the distal end of the liner (17),
- at least one electrode (25) electrically connected with the connection device (13) that is arranged adjacent to the end opening (30) or that extends beyond the end opening (30) in a longitudinal direction (L),
- a flushing channel (32) fluidically connected with the connection device (13) that comprises an exit opening (33) that opens out into a flow chamber (31) limited by the liner (17) and that is arranged with distance to the distally arranged end opening (30) in longitudinal direction (L), such that a flushing fluid (F) flowing through the flushing channel (32) flows out of the exit opening (33) and creates a flushing fluid flow (SF) in the flow chamber (31) from the exit opening (33) in direction toward the end opening (30), and
- a suction channel (34) fluidically connected with the connection device (13) that comprises an inlet opening (35) opening out into the flow chamber (31) and that is configured to receive at least a portion of the flushing fluid (F) of the flushing fluid flow (SF) via the inlet opening (35) and to discharge it from the flow chamber (31),
- a light conducting device (40) that comprises a light inlet surface (41),
**characterized in that** the light inlet surface (41) is arranged inside the liner (17), wherein at least the distal end section of the light conducting device (40) adjoining the light inlet surface (41) is arranged inside the flushing channel (32).

2. Electrosurgical instrument according to claim 1, wherein the inlet opening (35) of the suction channel (34) is arranged with distance to the end opening (30) in longitudinal direction (L).

3. Electrosurgical instrument according to any of the preceding claims, wherein the inlet opening (35) of the suction channel (34) is arranged closer to the end opening (30) than the exit opening (33) of the flushing channel (32) in longitudinal direction (L) or wherein the inlet opening (35) and the exit opening (33) have equal distances to the end opening (30) in longitudinal direction (L).

4. Electrosurgical instrument according to any of the preceding claims, wherein the light inlet surface (41) of the light conducting device (40) is further away from the end opening (30) in longitudinal direction (L) than the exit opening (33).

5. Electrosurgical instrument according to any of the preceding claims, wherein at least one sensor (55) electrically connected with the connection device (13) is comprised in the suction channel (34) and is configured to detect a pressure and/or a flow parameter inside the flushing channel (32).

6. Electrosurgical instrument according to any of the preceding claims, wherein the liner (17) is part of a housing (16) on which the connection device (13) is arranged on the proximal end.

7. Electrosurgical instrument according to any of the claims 1 to 5 that is configured as flexible endoscopic or laparoscopic instrument.

8. Electrosurgical instrument according to claim 7, wherein the liner (17) comprises a flexible hose (45) and/or a rigid tube (46).

9. Electrosurgical device (10) comprising an electrosurgical instrument (11) according to any of the preceding claims, a supply apparatus (12) that is connected with the electrosurgical instrument (11) by means of the connection device (13), and a light analysis unit (21)
wherein the light analysis unit (21) is provided that is optically connected with the light conducting device (40) and that is configured to carry out an analysis of the light received via the light inlet surface (41), wherein the supply apparatus (12) comprises a voltage source (18) that is electrically connected with the at least one electrode (25) of the electrosurgical instrument (11) in order to apply an electrical voltage potential to the at least one electrode (25),
wherein the supply apparatus (12) comprises a flushing fluid source (19) that is fluidically connected with the flushing channel (32) in order to supply flushing fluid (F) to the flushing channel (32),
and wherein the supply apparatus (12) comprises a suction unit (20) that is fluidically connected with the suction channel (34) of the electrosurgical instrument (11) in order to discharge at least a portion of flushing fluid (F) of the flushing fluid flow (SF) from the flow chamber (31) via the inlet opening (35) and the suction channel (34).

10. Electrosurgical device according to claim 9, wherein the supply apparatus (12) is configured to adjust a volume flow rate of the flushing fluid (F) by means of the flushing fluid source (19) and to adjust a volume flow rate of the suction flow by means of the suction unit (20), wherein the volume flow rate of the suction flow is at least as large as the volume flow rate of the flushing fluid.

11. Electrosurgical device according to claim 9 or 10, wherein the supply apparatus (12) is configured to adjust a volume flow rate of the suction flow and/or a suction power by means of the suction unit (20) such that no flushing fluid (F) exits from the end opening (30) of the liner (17).

12. Electrosurgical device according to any of the claims 9 to 11, wherein the supply apparatus (12) is configured to adjust a volume flow rate of the suction flow and/or a suction power by means of the suction unit (20) that fumes created during treatment of tissue (26) can be sucked through the end opening (30) of the liner (17).

## Revendications

1. Instrument électrochirurgical (11) comprenant :
- un dispositif de raccordement (13) conçu en vue d'une connexion à un appareil d'alimentation (12),
- une enveloppe (17) comportant une ouverture d'extrémité (30) située à l'extrémité distale de l'enveloppe (17),
- au moins une électrode (25) qui est reliée électriquement au dispositif de raccordement (13) et est placée à proximité de l'ouverture d'extrémité (30) ou dépasse dans une direction longitudinale (L) par rapport à l'ouverture d'extrémité (30),
- un conduit de rinçage (32) qui est relié du point de vue fluidique au dispositif de raccordement (13) et présente une ouverture de sortie (33) qui débouche dans un espace d'écoulement (31) délimité par l'enveloppe (17) et qui est disposée à distance de l'ouverture d'extrémité (30) distale, dans la direction longitudinale (L), de sorte qu'un fluide de rinçage (F) s'écoulant dans le conduit de rinçage (32) sort par l'ouverture de sortie (33) et génère dans l'espace d'écoulement (31) un courant de fluide de rinçage (SF), depuis l'ouverture de sortie (33) en direction de l'ouverture d'extrémité (30) , et
- un conduit d'aspiration (34) qui est relié du point de vue fluidique au dispositif de raccordement (13) et présente une ouverture d'entrée (35) débouchant dans l'espace d'écoulement (31), et qui est conçu pour accueillir au moins une partie du fluide de rinçage (F) du courant de fluide de rinçage (SF), par l'intermédiaire de l'ouverture d'entrée (35), et l'évacuer de l'espace d'écoulement (31),
- un dispositif de guidage de lumière (40) qui présente une surface d'entrée de lumière (41),
**caractérisé en ce que** le dispositif de guidage de lumière (40) est placé à l'intérieur de l'enveloppe (17), sachant qu'au moins la partie d'extrémité distale du dispositif de guidage de lumière (40) qui se situe à la suite de la surface d'entrée de lumière (41) est disposée dans le conduit de rinçage (32).

2. Instrument électrochirurgical selon la revendication 1, dans lequel l'ouverture d'entrée (35) du conduit d'aspiration (34) est disposée à distance de l'ouverture d'extrémité (30), dans la direction longitudinale (L).

3. Instrument électrochirurgical selon une des revendications précédentes, dans lequel l'ouverture d'entrée (35) du conduit d'aspiration (34) est disposée, dans la direction longitudinale (L), plus près de l'ouverture d'extrémité (30) que l'ouverture de sortie (33) du conduit de rinçage (32), ou dans lequel l'ouverture d'entrée (35) et l'ouverture de sortie (33) présentent la même distance par rapport à l'ouverture d'extrémité (30), dans la direction longitudinale (L).

4. Instrument électrochirurgical selon une des revendications précédentes, dans lequel la surface d'entrée de lumière (41) du dispositif de guidage de lumière (40) est plus éloignée de l'ouverture d'extrémité (30) que l'ouverture de sortie (33), dans la direction longitudinale (L).

5. Instrument électrochirurgical selon une des revendications précédentes, dans lequel il est prévu dans le conduit d'aspiration (34), au moins un capteur (55) qui est relié électriquement au dispositif de raccordement (13) et qui est conçu pour détecter une pression et/ou un paramètre d'écoulement dans le conduit d'aspiration (32).

6. Instrument électrochirurgical selon une des revendications précédentes, dans lequel l'enveloppe (17) fait partie d'un boîtier (16) sur lequel le dispositif de raccordement (13) est disposé à l'extrémité proximale.

7. Instrument électrochirurgical selon une des revendications 1 à 5, qui est réalisé comme instrument endoscopique souple ou laparos-copique.

8. Instrument électrochirurgical selon la revendication 7, dans lequel l'enveloppe (17) présente un tuyau flexible (45) et/ou un tube rigide (46).

9. Dispositif électrochirurgical (10) comprenant un instrument électrochirurgical (11) selon une des revendications précédentes, un appareil d'alimentation (12), qui est relié à l'instrument électrochirurgical (11) au moyen du dispositif de raccordement (13), et une unité d'analyse de lumière (21),
l'unité d'analyse de lumière (21) étant présente et étant reliée optiquement au dispositif de guidage de lumière (40) et étant conçue pour réaliser une analyse de la lumière reçue par l'intermédiaire de la surface d'entrée de lumière (41),
l'appareil d'alimentation (12) présentant une source de tension (19) qui est reliée électriquement à l'électrode (25), au nombre d'au moins une, de l'instrument électrochirurgical (11), aux fins d'appliquer un potentiel de tension électrique à l'électrode (25), au nombre d'au moins une,
l'appareil d'alimentation (12) présentant une source de fluide de rinçage (19) qui est reliée du point de vue fluidique au conduit de rinçage (32), aux fins d'amener le fluide de rinçage (F) au conduit de rinçage (32),
et l'appareil d'alimentation (12) présentant une unité d'aspiration (20) qui est reliée du point de vue fluidique au conduit d'aspiration (34) de l'instrument électrochirurgical (11), aux fins d'évacuer de l'espace d'écoulement (31) au moins une partie du fluide de rinçage (F) du courant de fluide de rinçage (SF), par l'intermédiaire de l'ouverture d'entrée (35) et du conduit d'aspiration (34).

10. Dispositif électrochirurgical selon la revendication 9, dans lequel l'appareil d'alimentation (12) est conçu pour régler un débit volumique du fluide de rinçage (F), au moyen de la source de fluide de rinçage (19), et pour régler un débit volumique du courant d'aspiration, au moyen de l'unité d'aspiration (20), le débit volumique du courant d'aspiration étant au moins égal au débit volumique du fluide de rinçage.

11. Dispositif électrochirurgical selon la revendication 9 ou 10, dans lequel l'appareil d'alimentation (12) est conçu pour régler, au moyen de l'unité d'aspiration (20), un débit volumique du courant d'aspiration et/ou une puissance d'aspiration, de manière à ce qu'aucun fluide de rinçage (F) ne sorte par l'ouverture d'extrémité (30) de l'enveloppe (17).

12. Dispositif électrochirurgical selon une des revendications 9 à 11, dans lequel l'appareil d'alimentation (12) est conçu pour régler, au moyen de l'unité d'aspiration (20), un débit volumique du courant d'aspiration et/ou une puissance d'aspiration, de manière à ce que les fumées générées lors du traitement de tissus (26) soient aspirées à travers l'ouverture d'extrémité (30) de l'enveloppe (17).
